# EUROPEAN PATENT APPLICATION

(11) **EP 2 017 271 A1**
(43) Date of publication of application: **21.01.2009**
(21) Application number: 08011986.0
(22) Date of filing: 03.07.2008
(51) Int. Cl.: C07D 307/87

(54) **Process for the preparation of escitalopram**

(30) Priority: 06.07.2007 IN MA14642007
(71) Applicant: Aurobindo Pharma Limited, Hyderabad 500 038 (IN)
(72) Inventor: Vipin, Kumar Kaushik, Ameerpet Hyderabad 500 038 (IN); Umar, Khan Mohammed, Ameerpet Hyderabad 500 038 (IN); Narsimha, Reddy Bobbali, Ameerpet Hyderabad 500 038 (IN); Ranjith, Kumar Srinivasan, Ameerpet Hyderabad 500 038 (IN); Ramesh, Dandala, Ameerpet Hyderabad 500 038 (IN); Sivakumaran, Meenakshisunderam, Ameerpet Hyderabad 500 038 (IN)
(74) Representative: Wittkopp, Alexander

(57) **Abstract**

The present invention relates to a novel process for the preparation of Escitalopram, which comprises:
(ii) de-methylating (±)-1-[3-(dimethylamino)propyl]-1-(4-fluorophenyl)-1,3-dihydro-5-isobenzofurancarbonitrile (Formula II) (Citalopram) 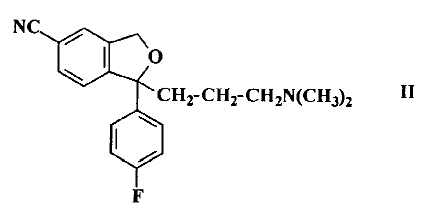 to produce (±)-1-[3-(methylamino)propyl]-1-(4-fluorophenyl)-1,3-dihydro-5-isobenzofurancarbonitrile (desmethyl Citalopram) (XII),
(iii) isolating the pure desmethyl Citalopram (XII),
(iv) separating the enantiomers from the pure desmethyl Citalopram (XII) with an optically active acid to obtain (*S*)-(+)-1-[3-(methylamino)propyl]-1-(4-fluorophenyl)-1,3-dihydro-5-isobenzofurancarbonitrile ((*S*)-(+)-desmethyl Citalopram) (XIII),
(v) methylating an enantiomerically pure compound (XIII) using suitable methylating agent to produce Escitalopram (I).

## Description

### FIELD OF THE INVENTION

The present invention relates to an industrially advantageous process for the preparation of pure Escitalopram, (*S*)-(+)-1-[3-(dimethylamino)propyl]-1-(4-fluorophenyl)-1,3-dihydro-5-isobenzofurancarbonitrile of Formula I, and its pharmaceutical acceptable salts.

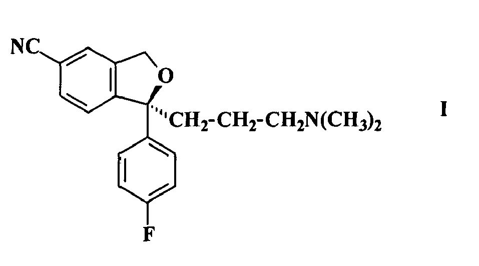

### BACKGROUND OF THE INVENTION

Escitalopram is the S-enantiomer of the antidepressant drug Citalopram of Formula II.

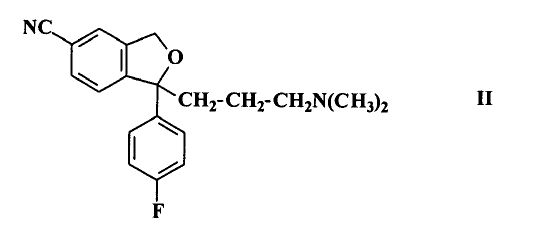

Citalopram is a well known antidepressant drug that has now been in the market for several years and is chemically known as 1-[3-(dimethylamino)propyl]-1-(4-fluorophenyl)-1,3-dihydroisobenzofuran-5-carbonitrile.

Citalopram is a selective centrally acting serotonin (5-HT) reuptake inhibitor. Citalopram was first disclosed in DE 2,657,013, corresponding to US 4,136,193. The antidepressant activity of Citalopram has been reported in several publications, e.g. J. Hyttel, Prog. Neuropsychopharmacol BioL Psychiat., 1982, 6 (3), 277-295 *and* A. Gravem, Acta Psychiatr. Scand., 1987, 75, 478-486*.*

The process for the preparation of antidepressant Citalopram and its pharmaceutical properties were first disclosed in US 4,136,193. Citalopram was produced from the corresponding 5-bromo derivative by reaction with cuprous cyanide. Further, variants of this method are disclosed in PCT Publications, WO 00/13648 and WO 00/11926, wherein the exchange of 5-halogen or 5-CF₃-(CF₂)ₙ-SO₂-O- with a cyano group is achieved with a cyanide source such as KCN, NaCN or (R'₄N)CN, where R'₄ indicates four groups which may be the same or different and are selected from hydrogen and straight chain or branched C₁₋₆ alkane, in presence of palladium or nickel catalyst.

The diol, 4-[4-(dimethylamino)-1-(4'-fluorophenyl)-1-hydroxybutyl]-3-(hydroxymethyl)-benzonitrile (VI), and its use as an intermediate in the preparation of Citalopram has been disclosed in US 4,650,884. In this reference, 5-cyanophthalide of Formula III is reacted successively with *p*-fluorophenylmagnesium bromide and 3-(N,N-dimethyl-amino)-propylmagnesium chloride to get the compound of Formula VI and its further conversion to Citalopram base is achieved by reaction with 70% sulfuric acid.

The process is shown in scheme I:

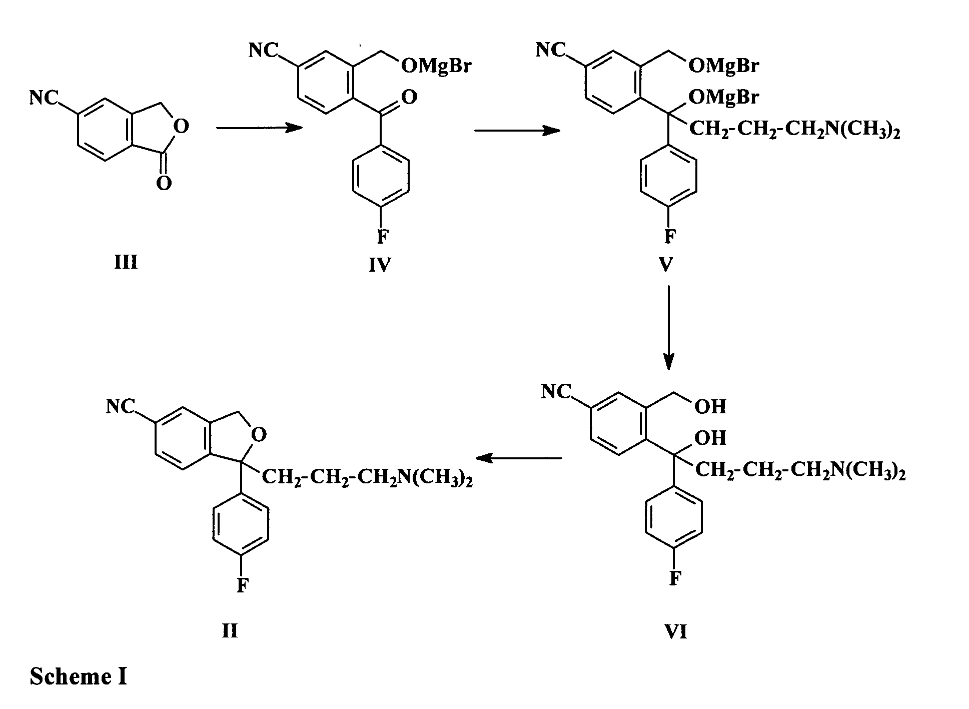

The S-enantiomer (Escitalopram) of Formula I and the antidepressant effect of said enantiomer is disclosed in US 4,943,590, wherein the use of Escitalopram for the treatment of neurotic disorders has been described. WO 02/087566 describes the use of Escitalopram for treating depressive patients who have failed to respond to conventional SSRIs.

Escitalopram has now been developed as an antidepressant and hence a need for a commercially feasible method to produce Escitalopram has emerged.

A process for the preparation of Escitalopram was first disclosed in US 4,943,590. According to this patent reference, attempts to resolve Citalopram enantiomers to produce Escitalopram were not successful. Therefore, resolution of enantiomers of the diol (VI) with optically active selective precipitant, di-*p*-toluoyl-D-tartaric acid, has been carried out to obtain (*S*)-enantiomer of the diol prior to ring closure in a stereospecific manner to obtain Escitalopram (I) as shown below:

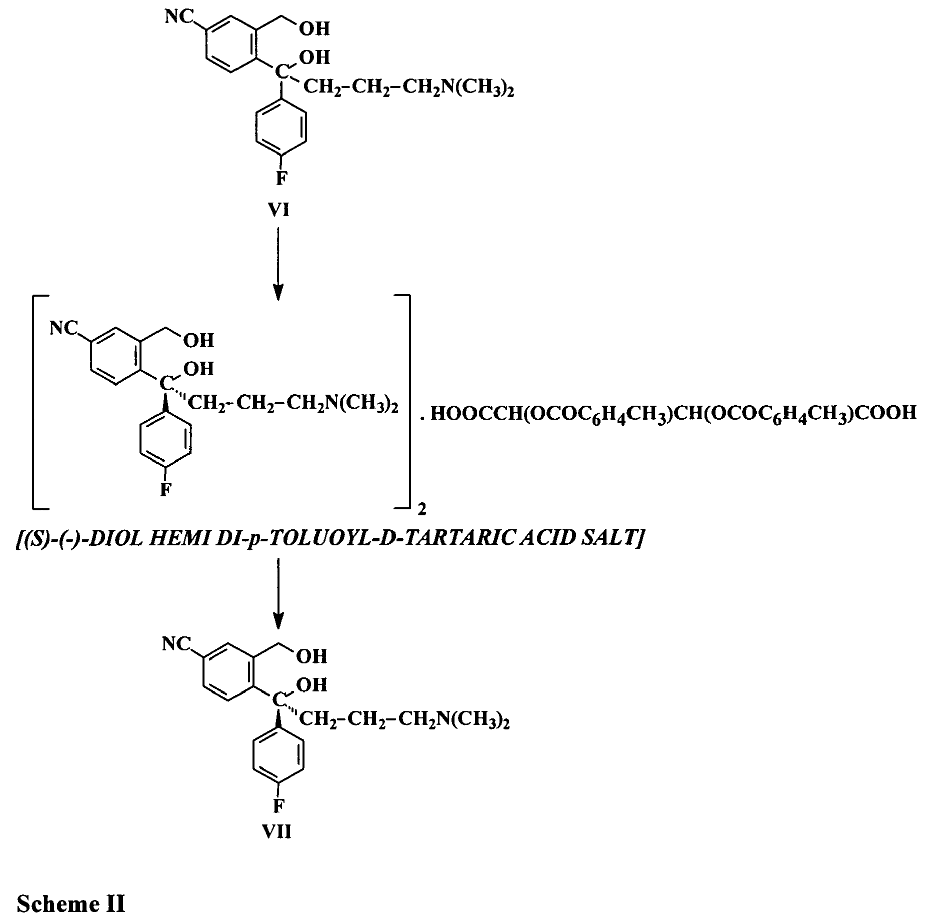

The resolution of enantiomers requires high purity of diol (VI) to selectively precipitate out (*S*)-diol hemi di-*p*-toluoyl-D-tartaric acid salt having substantially high chiral purity. The diol (VI), obtained as described in US 4,650,884, is not sufficiently pure and extensive purification steps have been described in this reference, which involve repeated charcoal and silica gel treatment of the diol (VI). Further, purification of diol (VI) has been carried out by preparing the hydrobromide salt and subsequently by crystallization, first from water and thereafter from 2-propanol/ethanol.

PCT publication WO 03/006449 discloses a process for the preparation of Escitalopram, involving chromatographic separation of the enantiomers of Citalopram and intermediates using a chiral stationary phase. The chiral stationary phase has to be found by screening the available chiral stationary phases for one, which is effective in separating the pair of enantiomers in question, and there may not always be an available chiral stationary phase suitable for the separation of the enantiomers. Further, chromatography is a batch process and involves the use of large quantities of solvents, and hence it is not suitable for industrial scale operations.

PCT Publication WO 03/087081 describes a process for the preparation of Escitalopram via (4-bromo-2-(hydroxymethyl)phenyl)-(4-flourophenyl)methanol, where the racemic diol is converted to an enantiomerically enriched form by first converting the diol into the monoester intermediate and then reacting the monoester intermediate with an optically active acid to form the diastereomeric salt. This salt is then crystallized to obtain an enantiomerically enriched S-isomer, whereupon the monoester intermediate is further converted to Escitalopram through suitable chemical conversions. The major drawbacks of the described process are low yields, usage of the hazardous material copper cyanide, and a lengthy process of production.

PCT Publication WO 03/051861 describes the separation of racemic bromo-Citalopram to the corresponding S-bromo-Citalopram by fractional crystallization of the diastereomeric salt of bromo-Citalopram, followed by hydrolysis and cyanation to obtain Escitalopram. The major drawback of this process is the use of cyanide in the presence of a palladium or nickel catalyst for the conversion of bromo to cyano groups, which is industrially not desirable due to safety concerns. This document also describes the separation of the bromodiol intermediate by chromatography using a chiral stationary phase, which is industrially not feasible.

PCT Publication WO 05/047274 describes a process for the preparation of Escitalopram by reacting 5-cyano-1-(4-fluorophenyl)-1,3-dihydroisobenzofuran (VIII) with 3-chloropropylamine (IX) in the presence of a base to produce racemic 1-(3-aminopropyl)-1-(4-fluorophenyl)-1,3-dihydro-5-isobenzofurancarbonitrile (X). The racemic compound (X) is treated with an enantiomerically pure acid followed by hydrolysis to produce the pure enantiomer S-(+)-1-(3-aminopropyl)-1-(4-fluorophenyl)-1,3-dihydro-5-isobenzofuran-carbonitrile (XI), methylation of which using suitable methylating agents yields Escitalopram (I). The process is as shown in Scheme III:

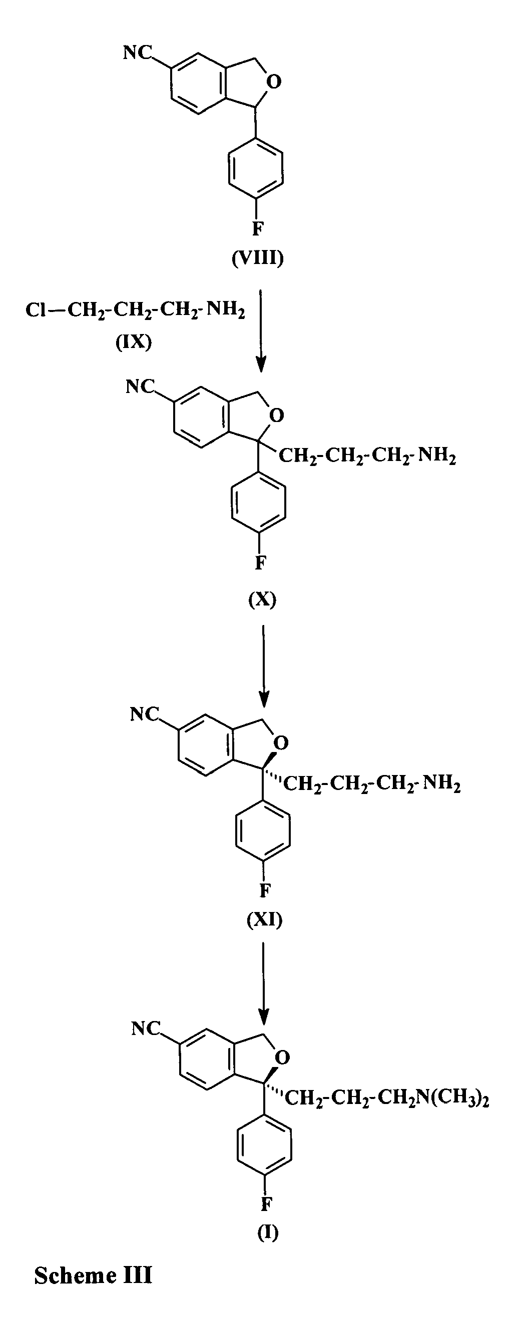

There is still a need for a cost effective, safe and industrially feasible process for the preparation of Escitalopram and the present invention is directed towards a novel process for the preparation of Escitalopram, which is cost effective, suitable on a large scale, with high yield and widely available chemicals.

### OBJECTIVE OF THE INVENTION

The main objective of the present invention is to provide a simple and effective process for the preparation of Escitalopram with high purity and yield on a commercial scale.

### SUMMARY OF THE INVENTION

Accordingly, the present invention provides a process for the preparation of (*S*)-(+)-1-[3-(dimethylamino)propyl]-1-(4-fluorophenyl)-1,3-dihydro-5-isobenzofurancarbonitrile (Escitalopram) of Formula I, and its salt, 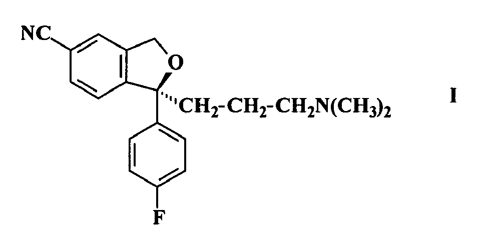 which comprises:
(i) de-methylating (±)-1-[3-(dimethylamino)propyl]-1-(4-fluorophenyl)-1,3-dihydro-5-isobenzofurancarbonitrile (Formula II) 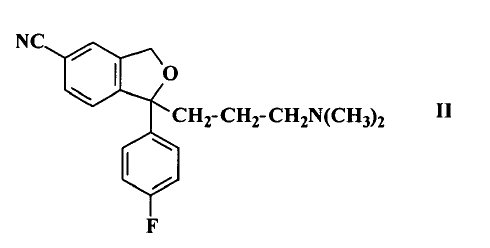 to produce (±)-1-[3-(methylamino)propyl]-1-(4-fluorophenyl)-1,3-dihydro-5-isobenzofurancarbonitrile (desmethyl Citalopram) (XII) 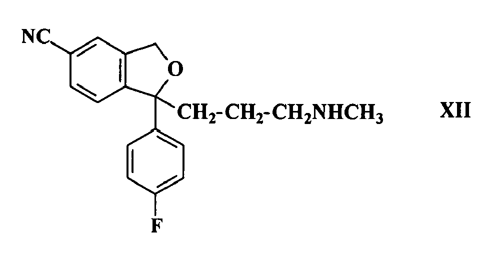
(ii) isolating the pure desmethyl Citalopram (XII)
(iii) separating the enantiomers from the pure desmethyl Citalopram (XII) with an optically active acid to obtain (*S*)-(+)-1-[3-(methylamino)propyl]-1-(4-fluorophenyl)-1,3-dihydro-5-isobenzofurancarbonitrile ((*S*)-(+)-desmethyl Citalopram) (XIII) 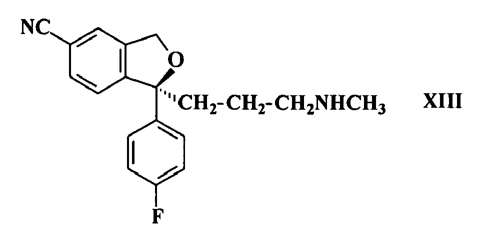
(iv) methylating an enantiomerically pure compound (XIII) using suitable methylating agent to produce Escitalopram (I).

### DETAILED DESCRIPTION OF THE INVENTION

The present invention relates to a novel process for the preparation of (*S*)-(+)-1-[3-(dimethylamino)propyl]-1-(4-fluorophenyl)-1,3-dihydro-5-isobenzofurancarbonitrile-(Escitalopram) of Formula I.

(±)-1-[3-(Dimethylamino)propyl]-1-(4-fluorophenyl)-1,3-dihydro-5-isobenzofuran-carbonitrile (Citalopram) (II), used as a starting material in the process of the present invention, is synthesized from 5-cyanophthalide by two successive Grignard reactions with 4-fluorophenylmagnesium bromide and 3-(*N*,*N*-dimethylamino)propylmagnesium chloride to produce Diol (VI), which is cyclised to produce Citalopram.

According to the present invention, demethylation of (±)-1-[3-(dimethylamino)-propyl]-1-(4-fluorophenyl)-1,3-dihydro-5-isobenzofurancarbonitrile (Citalopram) (II) is carried out using haloformates selected from 1-chloroethyl chloroformate, 1 chloromethyl chloroformate, phenyl chloroformate, ethyl chloroformate, and benzoyl chloroformate; phosgene derivatives; carbonyl analogues and carbonates selected from dimethylcarbonate, diethylcarbonate and mixtures thereof in an organic solvent selected from ethylene dichloride, methylene chloride, propylene chloride, toluene, xylene, cyclohexane, and heptane; preferably ethylene dichloride. After completion of the reaction, the reaction mass is concentrated at about 40-100°C under reduced pressure and DM water is added to the residue at 20-25°C and the product is extracted into organic solvent selected from toluene or ethyl acetate. The organic layer is concentrated at about 50-55°C under reduced pressure. The obtained residue is treated with an aqueous acid, an alkaline solution or an alcoholic solution such as ethanol, methanol and isopropanol at temperature of 40-100°C to produce desmethyl Citalopram (XII).

In an alternative method desmethyl Citalopram (XII) can also be prepared by reacting 1(4-fluorophenyl)-1,3-dihydro-5-isobenzofurancarbonitrile with a (3-bromo-propoxy)-tert-butyldimethylsilane in the presence of a base selected from LDA (lithiumdiisopropylamine), LiHMDS (hexamethyldisilasan lithium), NaH, NaHMDS (hexamethyldisilasan sodium) and metalalkoxides such as NaOMe, KOMe, LiOMe, NaOtertBu, KOtertBu and LiOtertBu in an organic solvent such as THF (tetrahydro-furane), DMF (dimethylformamide) and NMP (N-methylpyrrolidone), at a temperature of about -60 to -80°C, followed by removal of the silyl protecting group to produce 1-(4-fluorophenyl)-1-(3-hydroxypropyl)-1,3-dihydro-5-isobenzofurancarbonitrile , which is further reacted with methanesulfonyl chloride in the presence of a base selected from triethylamine and diethylamine in a solvent selected from tetrahydrofuran and toluene to produce 1-(4-fluorophenyl)-1-[(3-methanesulfonyloxy)-propyl]-1,3-dihydro-5-isobenzofurancarbonitrile.1-(4-Fluorophenyl)-1-[(3-methane-sulfonyloxy)-propyl]-1,3-dihydro-5-isobenzofurancarbonitrile is further reacted with methylamine in a solvent selected from methanol, ethanol, isopropanol, tetrahydrofuran and mixtures thereof to produce desmethyl Citalopram (XII).

In an another embodiment of the present invention, desmethyl Citalopram (XII) obtained from the above methods, is optionally purified by adding DM water to the desmethyl Citalopram (XII) and washed with an organic solvent selected from toluene, ethyl acetate, chloroform, and methylene chloride; preferably in toluene to remove undesired impurities, followed by adjusting the pH of the aqueous layer containing pure desmethyl Citalopram (XII) to a value of about 9 to 9.5 using a base such as aqueous ammonia, sodium hydroxide and sodium bicarbonate. The compound is extracted from the basified aqueous layer with a suitable organic solvent selected from toluene, ethyl acetate, chloroform, and methylene chloride; preferably in toluene and distilling the solvent to produce pure desmethyl Citalopram (XII).

In an another embodiment of the present invention, desmethyl Citalopram (XII) is optionally converted into its corresponding acid addition salt such as hydrochloride, hydrobromide, and oxalate by treating with a suitable acidic reagent in a suitable organic solvent. The salt of Compound (XII) is hydrolyzed in the presence of a base in a water and organic solvent mixture to produce pure desmethyl Citalopram (XII).

Pure (±)-1-[3-(methylamino)propyl]-1-(4-fluorophenyl)-1,3-dihydro-5-isobenzo-furancarbonitrile is treated with an optically active acid selected from dibenzoyl tartaric acid, bisnaphthylphosphoric acid, 10-camphorsulphonic acid, and di-(*p-*toluoyl)tartaric acid, in a solvent selected from toluene, an alcohol such as methanol, ethanol, isopropanol, butanol and mixtures thereof to resolve desmethyl Citalopram enantiomers to obtain (*S*)-(+)-1-[3-(methylamino)propyl]-1-(4-fluorophenyl)-1,3-dihydro-5-isobenzofurancarbonit- rile (XIII). Preferably di-*p*-toluoyl-D-tartaric acid is used to obtain (*S*)-enantiomer of desmethyl Citalopram (XII) having HPLC chiral purity of more than 98 %.

(*S*)-(+)-1-[3-(Methylamino)propyl]-1-(4-fluorophenyl)-1,3-dihydro-5-isobenzofuran-carbonitrile (XIII) is reacted with a methylating reagent selected from methyl iodide, dimethyl sulfate, and a mixture of formic acid and formaldehyde more preferably with the mixture of formic acid and formaldehyde at a temperature of about 80-95°C to produce Escitalopram (I). Optionally methylation is carried out in a solvent selected from toluene, xylene and ethylene dichloride. After completion of the reaction, the reaction mass is concentrated to residue. DM water and toluene is added to the obtained residue and the pH of the aqueous layer is adjusted to 9 to 9.5 using aqueous ammonia and the product is extracted with toluene and the toluene layer is concentrated to produce Escitalopram base.

Escitalopram base thus obtained is dissolved in an organic solvent selected from acetone, acetonitrile, ethanol, methanol, isopropanol, tetrahydrofuran, toluene, cyclohexane, and isopropyl ether; preferably in acetone and is treated with an oxalic acid dihydrate to obtain Escitalopram oxalate, which is isolated and dried by conventional methods. This process of the present invention provides Escitalopram oxalate with HPLC purity more than 99.5 %.

The details of the process of the invention are provided in the Examples given below which are provided by way of illustration only and therefore should not be construed to limit the scope of the invention.

### EXAMPLE: 1

### Stage I: PREPARATION OF PURE [1-(4-FLUOROPHENYL)-1-(3-METHYLAMINOPROPYL)-1,3-DIHYDROISOBENZOFURAN-5-CARBONITRILE HYDROCHLORIDE (DESMETHYL CITALOPRAM HYDROCHLORIDE)

### Step A:

Citalopram base (150 g, 0.46 mol) was dissolved in ethylene dichloride (750 ml) and chloromethyl chloroformate (89.54 g, 0.69 mol) was added at 0-5°C. The contents were heated slowly to 85-90°C and stirred at the same temperature to complete the reaction. After completion of the reaction, the reaction mass was concentrated at 50-55°C under reduced pressure (starting at 200 mm of Hg and stepwise reduction down to 10 mm of Hg). Thereafter, DM water (300 ml) was added to the reaction mass at 20-25°C and the obtained product was extracted into toluene at the same temperature. The organic layer was concentrated at 50-55°C under reduced pressure (200-10 mm of Hg) till no more solvent distills. Methanol (450 ml) was added to the concentrated mass containing the product and heated the reaction mass to 60-65°C and stirred to complete the reaction (by HPLC). After completion, methanol was removed by distillation at 50-55°C under reduced pressure (200-10 mm of Hg). DM water (450 ml) was added to the concentrated mass at 20-35°C and washed it with toluene to remove non salt forming impurities. The pH of the aqueous layer was adjusted to 9.5 using aqueous ammonia solution at 20-35°C and thereafter, the product was extracted into toluene. Finally, the organic layer was concentrated at 50-55°C under reduced pressure (200-10 mm of Hg) to produce the titled compound, desmethyl Citalopram base. ¹H NMR (DMSO-d₆) δ ppm 1.17-1.31 (m, 2H), 2.17-2.20 (d, 5H), 2.35-2.40 (t, 2H), 5.10-5.21 (dd, 2H), 7.12-7.18 (t, 2H), 7.56-7.61 (q, 2H), 7.72-7.79 (q, 3H)

### Step-B:

Desmethyl Citalopram base was dissolved in an ethyl acetate (600ml) at 20-25°C and the pH was adjusted to 3.5 with aqueous hydrochloric acid. The reaction mass was stirred for 4 hrs at 10-15°C to complete the precipitation. The product was filtered and dried at 40-45°C under vacuum to yield 120 g of desmethyl Citalopram hydrochloride, with HPLC purity of 99.5%.
¹HNMR (DMSO-d₆) δ ppm 1.41-1.61 (m, 2H), 2.27-2.33 (t, 2H), 2.35-2.38 (d, 3H) 2.82-2.87 (t, 2H), 5.15-5.27 (dd, 2H), 7.16-7.22 (t, 2H), 7.61-7.66 (q, 2H), 7.78-7.83 (t, 3H), 9.07 (s, 2H)

### Stage II: PREPARATION OF (S)-(+)-1-(4-FLUOROPHENYL)-1-(3-METHYLAMINOPROPYL)-1,3-DIHYDROISOBENZOFURAN-5-CARBONITRILE, (-)-DI-p-TOLUOYL-D-TARTARIC ACID SALT [(S)-(+)-DESMETHYL DPTTA SALT]

Racemic desmethyl Citalopram (20 g, 0.0645 mol) was dissolved in methanol (200 ml) at 20-35°C. Di-*p*-toluoyl-D-tartaric acid (25 g, 0.0648 mol) was added and slowly cooled to 25-30°C and stirred for 12 hrs. The crystals formed in the reaction mixture were filtered and washed with methanol to obtain the titled product (Chiral purity >90%).

The above salt was suspended in methanol (140 ml) and heated to 55-60°C to obtain a clear solution. The resulting solution was cooled to 25-30°C and stirred for 15 hrs. The solids were filtered and washed with methanol and thereafter dried to yield (S)-(+)-Desmethyl DPTTA salt
HPLC Purity: 99.83%
Chiral purity (by HPLC): 99.04% of Free Base
[α]_{D}²⁵ (of Free Base) (+) 12.5° (c₁ =1,in methanol)
¹H NMR (DMSO-d₆) δ ppm 1.330 (m, 2H), 2.135-2.182 (t, 2H), 2.358 (s, 9H), 2.724 (t, 2H), 5.101-5.209 (dd, 2H), 5,645 (s, 2H), 7.127-7.185 (t, 2H), 7.297-7.323 (d, 4H), 7.532-7.577 (t, 2H), 7.672-7.775 (dd, 3H), 7.840-7.866 (d, 4H)
MASS (PESCIEX-API 2000) ESI in +ve ion mode: m/z, 311.2 [(MH)⁺]

### Stage III: PREPARATION OF ESCITALOPRAM OXALATE

### Step A:

(S)-(+)-Desmethyl DPTTA salt (2.5 g, 0.00359 mol) was suspended in a mixture of

DM water (25 ml) and toluene (25 ml) at 25-30°C. The pH of the resulting solution was adjusted to 10 using aqueous sodium hydroxide solution at 25-30°C. The organic layer was separated, washed and partially concentrated at 50-55°C under reduced pressure to produce S-(+)-desmethyl Citalopram base as a residue.
¹H NMR (DMSO-d₆) δ ppm 1.138-1.337 (m, 2H), 2.169-2.257 (t, 5H), 2.358-2.404 (t, 2H), 5.108-5.214 (dd, 2H), 7.125-7.184 (t, 2H), 7.564-7.611 (q, 2H), 7.726-7.790 (q, 3H)
MASS (PESCIEX-API 2000) ESI in +ve ion mode: m/z, 311.2 [(MH)⁺]

### Step B:

Formic acid (0.45 g, 0.0097 mol) and formaldehyde (0.78 g, 0.026 mol) were added to the above obtained residue containing S-(+)-desmethyl Citalopram and heated to 90-95°C for 2 hrs. After completion of the reaction, the reaction mass was concentrated at 50-55°C under reduced pressure. 2N Hydrochloric acid (5 ml), toluene, and methylene chloride were added to the concentrated mass at 25-30°C and separated the organic layer. The pH of the aqueous layer was adjusted to 9.5 using aqueous ammonia solution at 20-35°C and thereafter, the product was extracted with methylene chloride. Finally, the organic layer was concentrated at 50-55°C (200-10 mm of Hg) to obtain Escitalopram base.
The oxalate salt of the above base was obtained by treating it with oxalic acid dihydrate in acetone.

## Claims

1. An improved process for the preparation of (*S*)-(+)-1-[3-(dimethyl-amino)propyl]-1 -(4-fluorophenyl)-1,3-dihydro-5-isobenzofurancarbomtrile (Escitalopram) of Formula I, and its salt 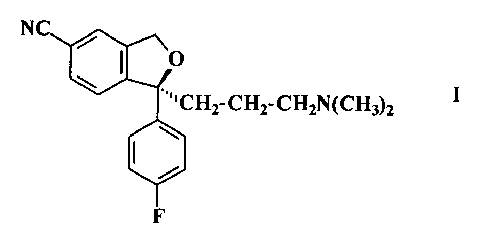 which comprises:
(i) demethylating (±)-1-[3-(dimethylamino)propyl]-1-(4-fluorophenyl)-1,3-dihydro-5-isobenzofurancarbonitrile (Citalopram) of Formula II 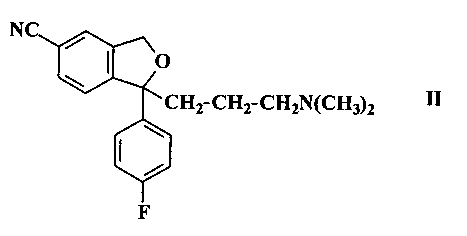 to produce (±)-1-[3-(methylammo)propyl]-1-(4-fluorophenyl)-1,3-dihydro-5-isobenzofurancarbonitrile (desmethyl Citalopram) of formula XII 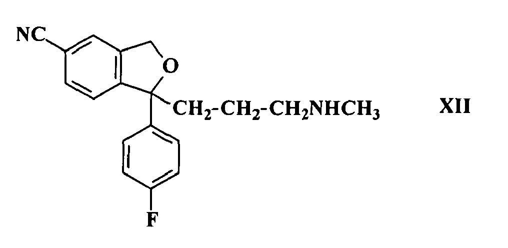
(ii) isolating the pure desmethyl Citalopram (XII)
(iii) separating the enantiomers of the pure desmethyl Citalopram (XII) using an optically active acid to obtain (*S*)-(+)-1-[3-(methylamino)-propyl]-1-(4-fluorophenyl)-1,3-dihydro-5-isobenzofurancarbonitrile ((*S*)-(+)-desmethyl Citalopram) of formula XIII 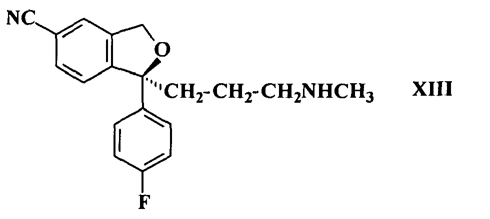
(iv) methylating the enantiomerically pure (*S*)-(+)-desmethyl Citalopram of formula XIII using suitable methylating agent to produce Escitalopram of formula I.

2. A process according to claim 1, wherein the demethylation is carried out in the presence of haloformates, phosgene derivatives, carbonyl analogues, carbonates or mixtures there of in an organic solvent.

3. A process according to claim 1 or claim 2, wherein the haloformate is selected from 1-chloroethyl chloroformate, 1-chloromethyl chloroformate, phenyl chloroformate, ethyl chloroformate, and benzoyl chloroformate.

4. A process according to any one of claims 1-3, wherein the carbonate is selected from dimethylcarbonate, diethylcarbonate, and mixture thereof.

5. A process according to any one of claims 1-4, wherein the organic solvent is selected from ethylene dichloride, methylene chloride, propylene chloride, toluene, xylene, cyclohexane, and heptane; preferably ethylene dichloride.

6. A process according to any one of claims 1-5, wherein the isolation of pure desmethyl Citalopram (XII) involves concentration of the reaction mass at about 40-100°C under reduced pressure, addition of DM water to the residue at 20-25°C, and extraction of the product into an organic solvent, followed by concentrating the organic layer at about 50-55°C under reduced pressure to produce a residue, which is treated with an alcoholic solution at a temperature of about 50-80°C, and removing the alcohol to produce desmethyl Citalopram (XII), which is further treated with a base and subsequently extracted with an extraction solvent and isolated to afford pure desmethyl Citalopram of formula XII.

7. A process according to any one of claims 1-6, wherein the organic solvent is selected from toluene or ethyl acetate.

8. A process according to any one of claims 1-7, wherein the alcohol is selected from methanol, ethanol, or isopropanol.

9. A process according to any one of claims 1-8, wherein the base is selected from aqueous ammonia, sodium hydroxide and sodium bicarbonate.

10. A process according to claim 6, wherein the extraction solvent is selected from toluene, ethyl acetate, chloroform, and methylene chloride; preferably toluene.

11. A process according to any one of claims 1-10, wherein the optically active acid is selected from dibenzoyl tartaric acid, bisnaphthylphosphoric acid, 10-camphorsulphonic acid, and di-(*p*-toluoyl)tartaric acid; preferably di-*p*-toluoyl-D-tartaric acid.

12. A process according to any one of claims 1-11, wherein the methylation is carried out with a methylating reagent selected from methyl iodide, and dimethyl sulfate.

13. A process according to any one of claims 1-11, wherein the methylation is carried out in a mixture of formic acid and formaldehyde.

14. A process according to any one of claims 1-13, wherein the methylation is carried out in the presence of a solvent selected from toluene, xylene, and ethylene dichloride.

15. Pure di-*p*-toluoyl-D-tartaric acid salt of (*S*)-(+)-1-[3-(methylamino)propyl]-1-(4-fluorophenyl)-1,3-dihydro-5-isobenzofurancarbonitrile of formula XIV.

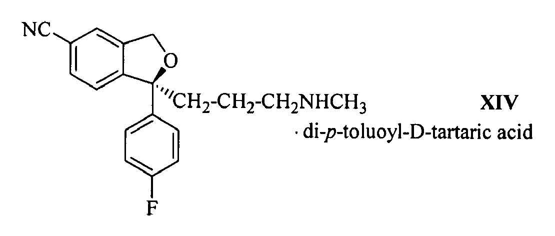

16. A process for the preparation of Escitalopram utilizing the di-*p*-toluoyl-D-tartaric acid salt of (*S*)-(+)-1-[3-(methylamino)propyl]-1-(4-fluorophenyl)-1,3-dihydro-5-isobenzofurancarbonitrile (XIV).
